(19) Europäisches Patentamt / European Patent Office / Office européen des brevets

(11) **EP 4 350 008 A1**

(12) **EUROPEAN PATENT APPLICATION**
published in accordance with Art. 153(4) EPC

(43) Date of publication:
**10.04.2024 Bulletin 2024/15**

(21) Application number: **22810256.2**

(22) Date of filing: **18.04.2022**

(51) International Patent Classification (IPC):
**C12Q 1/689** (2018.01)   **C12Q 1/06** (2006.01)
**G06F 17/18** (2006.01)

(52) Cooperative Patent Classification (CPC):
Y02A 50/30

(86) International application number:
**PCT/CN2022/087384**

(87) International publication number:
**WO 2022/247523 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.05.2021   CN 202110562717**

(71) Applicant: **Wedo (Suzhou) Biotechnological Co., Ltd**
**Suzhou, Jiangsu 215000 (CN)**

(72) Inventors:
- **CHEN, Peng**
  **Hangzhou, Zhejiang 311231 (CN)**
- **ZHOU, Zhongkun**
  **Hangzhou, Zhejiang 311231 (CN)**
- **LI, Yang**
  **Hangzhou, Zhejiang 311231 (CN)**
- **MA, Wantong**
  **Hangzhou, Zhejiang 311231 (CN)**
- **LIU, Yuheng**
  **Hangzhou, Zhejiang 311231 (CN)**
- **ZHANG, Rentao**
  **Hangzhou, Zhejiang 311231 (CN)**
- **MA, Yunhao**
  **Hangzhou, Zhejiang 311231 (CN)**
- **DU, Kangjia**
  **Hangzhou, Zhejiang 311231 (CN)**

(74) Representative: **SSM Sandmair**
**Patentanwälte Rechtsanwalt**
**Partnerschaft mbB**
**Joseph-Wild-Straße 20**
**81829 München (DE)**

Remarks:
The complete document including Reference Table(s) and the Sequence Listing(s) can be downloaded from the EPO website

(54) **BIOMARKER COMPOSITION FOR EARLY DIAGNOSIS OF COLORECTAL CANCER AND APPLICATION**

(57)     The present invention belongs to the field of colorectal cancer detection, and particularly relates to a biomarker combination for colorectal cancer early diagnosis and a use thereof. The present invention specifically discloses a use of the biomarker in colorectal cancer early diagnosis. The combination of a determination of the biomarker and a fecal occult blood test for the colorectal cancer early diagnosis has the advantages of low cost, convenience in detection, high accuracy, good sensitivity and strong specificity, and avoids pain, discomfort and complications caused by invasive colonoscopy, remarkably decreases the false positive rate of the colorectal cancer early diagnosis, and is able to be widely applied to the colorectal cancer early diagnosis.

**Description**

**TECHNICAL FIELD OF THE INVENTION** The present invention belongs to the field of colorectal cancer detection, and particularly relates to a biomarker combination for colorectal cancer early diagnosis and a use thereof.

**BACKGROUND OF THE INVENTION**

Colorectal cancer is a common clinical tumor featured with high incidence rate and mortality rate. In China, the incidence of colorectal cancer ranks fourth in men and third in women in terms of emerging cancer cases, and ranks second in the world. If colorectal cancer lesions can be detected and surgically excised in the early stage, the survival rate may exceed 90%, which decreases to 13% in the late stage. Therefore, early screening is the most effective means to reduce the mortality of colorectal cancer. Developed countries have established effective early screening plans and diagnostic techniques, which have became an important part of the method for selecting therapies and predicting results, thus greatly decreasing the mortality risk and improving treatment quality, and providing us with guidance and reference.

Regarding screening methods, colonoscopy is still the gold standard for definitive diagnosis. However, the effective colonoscopy may cause fear and embarrassment and lead to serious complications. Thus, the method is related to low participation rate and is not applicable to early screening. Other invasive screening methods, such as barium enema and magnetic resonance imaging, have the shortcomings of major technological hurdles and high cost. Non-invasive fecal occult blood tests include guaiacum fecal occult blood test (gFOBT) and fecal immunochemical test (FIT), which have the problems of low sensitivity and being susceptible to diet and medication. As recommend in *Guidelines for Screening of Early Colorectal Cancer,* subjects should have an annual check with the high-sensitivity gFOBT or the high-sensitivity FIT, but such methods have a high false positive rate and low sensitivity to polyps in early stage.

Fecal genes are another reliable biomarker, which can be used for early screening through DNA analysis. In the *Guidelines for Screening of Early Colorectal Cancer,* it is recommended to use the multi-target gene detection to screen the colorectal cancer once every three years. However, the method costs a lot and has a poor diagnostic effect on adenoma, and has not been validated in Asian populations. Approved by FDA, Cologuard is an effective molecular screening technology (gene mutation, methylation and occult blood testing), which combines the fecal occult blood detection with tumor-related DNA marker detection in blood to realize colorectal cancer early diagnosis by quantitatively detecting the DNA markers related to colorectal cancer in blood and occult haemoglobin in human feces. However, it is high-priced, requires blood sample and has poor diagnostic effect on adenoma. Therefore, we need to develop sensitive, non-invasive and economical non-invasive screening technologies to improve the present situation.

Studies have shown that intestinal microbiota plays an important role in human health and many diseases, so they can be used as biomarkers for diagnosis, prognosis, stratification and so forth, and can be detected in feces of patients. For example, the Chinese patent No. CN106574294A discloses a method, a primer and a kit for diagnosing the colorectal cancer from human fecal samples by quantitative PCR. The method detects the quantitative levels of one or more 16SrDNA bacterial sequences in feces of human subjects for CRC and/or adenomatous polyp early detection, risk screening and monitoring. However, the method has low accuracy and sensitivity in colorectal cancer detection. The Chinese patent CN110512015A discloses a biomarker combination for intestinal cancer and a use thereof. The biomarker combination includes Clostridiaceae, Porphyromonas, Peptostreptococcus, *Fusobacterium nucleatum* and Micromonas. The inventors collected a large number of fecal samples from patients with colorectal cancer and precancerosis (colorectal adenoma and colorectal neoplastic polyp) and healthy individuals, and conducted comparative analysis and validation of the differences in abundance and relative content of microorganisms in the fecal samples using high-throughput sequencing, real-time fluorescence quantitative PCR, high-resolution melting or biochip technique to determine the microorganisms related to the colorectal cancer and the precancerosis; and employed different combinations of the above mentioned microorganisms as the biomarker combinations, of which the abundances were detected in samples to predict the onset of the colorectal cancer. However, the method has poor detection specificity.

The present invention selects specific intestinal microorganisms as a biomarker combination for colorectal cancer early diagnosis. The biomarker combination includes *Peptostreptococcus stomatis, Fusobacterium nucleatum, Parvimonas micra* and *Bifidobacterium.* By combining the quantitative level detection of the microorganism combination with the fecal occult blood test, the present invention is used for colorectal cancer diagnosis through a regression equation. The present invention realizes the colorectal cancer early diagnosis utilizing specificity based on high-throughput sequencing results in combination with fecal occult blood test. It has advantages of high sensitivity and strong specificity, avoids pain, discomfort and complications caused by invasive colonoscopy, remarkably decreases the false positive rate of the colorectal cancer early diagnosis, and is able to be widely applied to the colorectal cancer early diagnosis.

## SUMMARY OF THE INVENTION

To overcome the above technical shortcomings, an object of the present invention is to provide a biomarker combination for colorectal cancer, which includes *Peptostreptococcus stomatis, Fusobacterium nucleatum, Parvimonas micra* and *Bifidobacterium.*

Another object of the present invention is to provide a kit for detecting colorectal cancer, which includes an assay reagent for a biomarker combination, and the biomarker combination comprises *Peptostreptococcus stomatis, Fusobacterium nucleatum, Parvimonas micra* and *Bifidobacterium.*

Preferably, the assay reagent is used for quantifying each component of the biomarker combination.

Preferably, the principle followed by the assay reagent is real-time quantitative PCR.

Preferably, the assay reagent is a real-time quantitative PCR assay reagent.

Preferably, the assay reagent comprises a primer group for the biomarker combination.

Preferably, the primer group comprises a primer pair for amplifying each component of the biomarker combination.

Preferably, the primer group has nucleotide sequences as shown in SEQ ID NO. 1-8.

Preferably, the assay reagent further comprises a primer pair for amplifying total bacteria.

Preferably, the primer pair for amplifying the total bacteria has nucleotide sequences as shown in SEQ ID NO.9-10.

Preferably, the assay reagent further comprises a real-time quantitative PCR reagent.

Preferably, the kit further comprises an occult blood test reagent for detecting human hemoglobin in feces.

Preferably, the occult blood test reagent comprises a dilution buffer and a test strip.

Another object of the present invention is to provide a use of the kit in colorectal cancer early diagnosis, wherein the colorectal cancer detection using the kit comprises the following steps: detecting an abundance and a fecal occult blood value of each component of a biomarker combination according to claim 1 in a sample, and then judging the detection result using a regression equation; and using genomic DNA from the feces of the subject as a template, detecting the abundance of the biomarkers in the sample with the primer group in the kit.

Preferably, colorectal cancer detection using the kit comprises the following steps:

(1) determination of the fecal occult blood value: determining the occult blood result value $X_0$ of the fecal sample of a subject using the fecal occult blood test kit, where $X_0 = 1$ when the occult blood result is positive and $X_0 = 0$ when the occult blood result is positive;

(2) using genomic DNA from the feces of the subject as a template, detecting $Ct_{marker}$, an amplified value of each biomarker and $Ct_{total\ bacteria}$, an amplified value of total bacteria with the primer group in the kit, separately;

(3) calculating a value Y according to a regression equation, and substituting the value Y into formula (II) to calculate a value P, where e is a natural constant,

$$P = \frac{e^Y}{1+e^Y}$$

$$(II);$$

(5) interpretation of result: when P>0.5, the diagnosis result of colorectal cancer is positive; and when P≤0.5, the diagnosis result of colorectal cancer is negative.

Preferably, the regression equation in step (4) is shown as formula (III),

$$Y = A + \beta_0 X_0 + \beta_1 X_1 + \beta_2 X_2 + \beta_3 X_3 + \beta_4 X_4$$

$$(III);$$

where, $X_0$ is the occult blood result value, $X_1$ is a Ct value of *Peptostreptococcus stomatis,* $X_2$ is a Ct value of *Fusobacterium nucleatum,* $X_3$ is a Ct value of *Parvimonas micra* and $X_4$ is a Ct value of *Bifidobacterium;* A and $\beta_0$-$\beta_4$ are constants, and A and $\beta_0$-$\beta_4$ are data obtained from clinical experiments.

Preferably, the regression equation in step (4) comprises any of formula (IV) to formula (VIII):

$$Y=-3.9583+3.1015X_0-0.6478X_1-0.3980X_2+0.2912X_3-0.5130X_4$$

$$(IV);$$

$$Y=-2.965+2.7005X_0-1.2309X_1-0.6054X_2+0.3328X_3-0.2359X_4$$

$$(V);$$

$$Y=-3.099+1.9907X_0-2.0011X_1-0.3290X_2+0.5476X_3-0.6620X_4$$

$$(VI);$$

$$Y=-3.771+4.0089X_0-0.9983X_1-0.4482X_2+0.1288X_3-0.4917X_4$$

$$(VII);$$

$$Y=-2.9043+2.8920X_0-0.5471X_1-0.1049X_2+0.2281X_3-0.6194X_4$$

$$(VIII).$$

Preferably, the regression equation in step (4) is:

$$Y=-2.965+2.7005X_0-1.2309X_1-0.6054X_2+0.3328X_3-0.2359X_4.$$

Preferably, the kit further comprises a DNA extraction reagent.

The beneficial effects of the present invention are as follows: ① the present invention provides a biomarker combination for colorectal cancer early diagnosis, and a primer pair and a kit for quantitative detection of the biomarker combination; ② the present invention realizes the colorectal cancer early diagnosis utilizing specificity based on high-throughput sequencing results in combination with fecal occult blood test. It has advantages of high sensitivity and strong specificity, avoids pain, discomfort and complications caused by invasive colonoscopy, remarkably decreases the false positive rate of the colorectal cancer early diagnosis, achieves non-invasive diagnosis of colorectal cancer and has a high value in widespread use.

## DETAILED DESCRIPTION OF THE INVENTION

The following examples are provided for thorough understanding of the present invention, but are not intended to limit the present invention. Unless otherwise specified, experimental methods applied in the following examples are conventional methods. Unless otherwise specified, experimental materials applied in the following examples are commercially available.

As used herein, the term "biomarker" refers to a disease marker as a substance that is normally present in body samples and is easy to be measured, and the measurement is correlated to the pathophysiology of underlying diseases, such as presence of CRC.

The term "quantification" refers to the capability of quantifying a specific nucleic acid sequence in a sample. Molecular biological methods for determining a target nucleic acid sequence include, but are not limited to, end point PCR, competitive PCR, reverse transcriptase PCR (PT-PCR), quantitative PCR (qPCR), PCR-ELISA and DNA microarray technique.

The term "quantification level" may be concentration (DNA amount per unit volume), DNA amount per cell, or cycle threshold (Ct value). In one preferred example, the quantification of the bacterial sequences is performed by qPCR; In one more preferred example, the quantification of the bacterial sequences is performed by qPCR, and the quantization level is expressed as a Ct value. The Ct value refers to the qPCR cycle number experienced when the fluorescence signal in each reaction tube reaches the preset threshold. The Ct level is inversely proportional to the quantification level of the target nucleic acid in the sample, to be specific, the lower the Ct level, the greater the target nucleic acid amount in the sample.

The abundance of the target nucleic acid sequence in the sample is quantified in an absolute manner or in a relative manner. The relative quantification is based on one or more internal control genes, namely, the 16S rRNA genes from the reference strain. For example, a universal primer is employed to determine the total bacterial count and the abundance of the target nucleic acid sequence is expressed as a percentage of total bacterial count. Absolute quantification is to obtain the exact number of target molecules by comparing with a DNA standard.

The term "primer" refers to an oligonucleotide that is used in an amplification method, such as a polymerase chain reaction (PCR), to amplify a nucleotide sequence. The primer is a polynucleotide sequence based on a specific target sequence.

In one specific example, sensitivity, specificity, accuracy and other combinations are used to describe the soundness and reliability of the present detection method. The terms used together with such descriptions as sensitivity, specificity and accuracy include: true positive (TP), true negative (TN), false positive (FP) and false negative (FN); where, if it is proved that the patient has a disease, and the given screening experiment also indicates the existence of the disease, the test result is considered to be true positive; if it is proved that the patient has no disease, and the given screening experiment also indicates the non-existence of the disease, the test result is considered to be true negative; If the results of the screening experiment indicate that the patient who does not actually have the disease has the disease, the test result is false positive; and If the results of the screening experiment indicate that the patient who actually has the disease does not have the disease, the test result is false negative.

$$\text{Sensitivity} = \text{TP/(TP+FN)} = \text{number of true positive cases/number of all positive cases;}$$

$$\text{Specificity} = \text{TN/(TN+FP)} = \text{number of true negative cases/number of all negative cases;}$$

$$\text{Accuracy} = \text{(TN+TP)/(TN+TP+FN+FP)} = \text{number of correct cases/number of all cases.}$$

Example 1: Design of primer group

Primer pairs are designed for *Peptostreptococcus stomatis, Fusobacterium nucleatum, Parvimonas micra, Bifidobacterium* and total bacteria, as shown in the following primer pair (1)-(5):

Primer pair (1): sense primer 5-AAGTGTTAGCGGTATAGGATG-3; anti-sense primer 5-CGTGTCTCAGTTCCAATGT-3;
Primer pair (2): sense primer 5-GGATTTATTGGGCGTAAAGC-3; anti-sense primer 5-GGCATTCCTACAAATATCTACGAA-3;
Primer pair (3): sense primer 5-GCGTAGATATTAGGAGGAATAC-3; anti-sense primer 5-GCGGAATGCTTAATGTGTT-3;
Primer pair (4): sense primer 5-CATCGCTTAACGGTGGAT-3-3; anti-sense primer 5-TTCGCCATTGGTGTTCTT-3;
Primer pair (5): sense primer 5-GCAGGCCTAACACATGCAAGTC-3; anti-sense primer 5-CTGCTGCCTCCCGTAGGAGT-3.

Example 2: Preparation and use method of the kit

2.1 Kit 1

A kit for detecting colorectal cancer, comprising an assay reagent for a biomarker combination, which includes *Peptostreptococcus stomatis, Fusobacterium nucleatum, Parvimonas micra* and *Bifidobacterium,* a reaction buffer system, Mix, ROX and RNase-free ddH$_2$O. The assay reagent includes a primer group for testing the biomarker combination, and the primer group is shown as the primer pairs (1) to (4) in Example 1. The assay reagent further includes a primer pair for testing total bacteria, and the primer pair is shown as the primer pair (5) in Example 1.

Use method: genomic DNA is extracted from feces of a subject to prepare a fecal genomic DNA template, and the abundances of the biomarkers in a sample are detected with the primer pairs (1) to (5). The existence of the colorectal cancer in the patient is determined according to the abundances of the biomarkers in the sample.

2.2 Kit 2

A kit for detecting colorectal cancer, the kit comprising an assay reagent for a biomarker combination, which includes *Peptostreptococcus stomatis, Fusobacterium nucleatum, Parvimonas micra* and *Bifidobacterium,* and an occult blood test reagent. The assay reagent includes a primer group, a reaction buffer system, Mix, ROX and RNase-free ddH$_2$O, and the primer group is shown as the primer pairs (1) to (4) in Example 1. The assay reagent further includes a primer pair for testing total bacteria, and the primer pair is shown as the primer pair (5) in Example 1. The occult blood test reagent includes a dilution buffer and a test strip.

Use method:

(1) determination of the fecal occult blood value: determining the occult blood result value $X_0$ of the fecal sample of a subject using the fecal occult blood test kit, where $X_0 = 1$ when the occult blood result is positive and $X_0 = 0$ when the occult blood result is positive;
(2) using genomic DNA from the feces of the subject as a template, detecting $Ct_{marker}$, an amplified value of each biomarker and $Ct_{total\ bacteria}$, an amplified value of total bacteria with the primer group in the kit, separately;
(3) calculating a value Y according to a regression equation $Y=A+\beta_0X_0+\beta_1X_1+\beta_2X_2+\beta_3X_3+\beta_4X_4$ (III), and substituting the value Y into formula (II) to calculate a value P, where e is a natural constant,

$$P= \frac{e^Y}{1+e^Y}$$

$$(II);$$

(4) interpretation of result: when P>0.5, the diagnosis result of colorectal cancer is positive; and when P≤0.5, the diagnosis result of colorectal cancer is negative.

Where, in the regression equation $Y=A+\beta_0X_0+\beta_1X_1+\beta_2X_2+\beta_3X_3+\beta_4X_4$ (III), $X_0$ is the occult blood result value, $X_1$ is a Ct value of *Peptostreptococcus stomatis,* $X_2$ is a Ct value of *Fusobacterium nucleatum,* $X_3$ is a Ct value of *Parvimonas micra,* and $X_4$ is a Ct value of *Bifidobacterium;* A and $\beta_0$-$\beta_4$ are constants and are obtained from analysis of clinical experiment data.

According to analysis of clinical trials, the regression equation is any of formula (IV) to formula (VIII):

$$Y=-3.9583+3.1015X_0-0.6478X_1-0.3980X_2+0.2912X_3-0.5130X_4$$

$$(IV);$$

$$Y=-2.965+2.7005X_0-1.2309X_1-0.6054X_2+0.3328X_3-0.2359X_4$$

$$(V);$$

$$Y=-3.099+1.9907X_0-2.0011X_1-0.3290X_2+0.5476X_3-0.6620X_4$$

$$(VI);$$

$$Y=-3.771+4.0089X_0-0.9983X_1-0.4482X_2+0.1288X_3-0.4917X_4$$

$$\text{(VII);}$$

$$Y=-2.9043+2.8920X_0-0.5471X_1-0.1049X_2+0.2281X_3-0.6194X_4$$

$$\text{(VIII).}$$

2.3 Kit 3

A kit for detecting colorectal cancer, comprising an assay reagent for a biomarker combination, which includes *Peptostreptococcus stomatis, Fusobacterium nucleatum, Parvimonas micra* and *Bifidobacterium,* an occult blood test reagent and a DNA extraction reagent. The assay reagent includes a primer group, a reaction buffer system, Mix, ROX and RNase-free ddE$_2$O, and the primer group is shown as the primer pairs (1) to (4) in Example 1. The assay reagent further includes a primer pair for testing total bacteria, and the primer pair is shown as the primer pair (5) in Example 1. The kit further includes; The occult blood test reagent includes a dilution buffer and a test strip.

Use method:

(1) determination of the fecal occult blood value: determining the occult blood result value $X_0$ of the fecal sample of a subject using the fecal occult blood test kit, where $X_0 = 1$ when the occult blood result is positive and $X_0 = 0$ when the occult blood result is positive;
(2) using genomic DNA from the feces of the subject as a template, detecting $Ct_{marker}$, an amplified value of each biomarker and $Ct_{total\ bacteria}$, an amplified value of total bacteria with the primer group in the kit, separately;
(3) calculating a value Y according to a regression equation $Y=A+\beta_0X_0+\beta_1X_1+\beta_2X_2+\beta_3X_3+\beta_4X_4$ (III), and substituting the value Y into formula (II) to calculate a value P, where e is a natural constant,

$$P=\frac{e^Y}{1+e^Y}$$

$$\text{(II);}$$

(4) interpretation of result: when P>0.5, the diagnosis result of colorectal cancer is positive; and when P≤0.5, the diagnosis result of colorectal cancer is negative.

Where, in the regression equation $Y=A+\beta_0X_0+\beta_1X_1+\beta_2X_2+\beta_3X_3+\beta_4X_4$ (III), $X_0$ is the occult blood result value, $X_1$ is a Ct value of *Peptostreptococcus stomatis,* $X_2$ is a Ct value of *Fusobacterium nucleatum,* $X_3$ is a Ct value of *Parvimonas micra,* and $X_4$ is a Ct value of *Bifidobacterium;* A and $\beta_0$-$\beta_4$ are constants and are obtained from analysis of clinical experiment data.

According to analysis of clinical trials, the regression equation is any of formula (IV) to formula (VIII):

$$Y=-3.9583+3.1015X_0-0.6478X_1-0.3980X_2+0.2912X_3-0.5130X_4$$

$$\text{(IV);}$$

$$Y=-2.965+2.7005X_0-1.2309X_1-0.6054X_2+0.3328X_3-0.2359X_4$$

$$\text{(V);}$$

$$Y=-3.099+1.9907X_0-2.0011X_1-0.3290X_2+0.5476X_3-0.6620X_4$$

$$(VI);$$

$$Y=-3.771+4.0089X_0-0.9983X_1-0.4482X_2+0.1288X_3-0.4917X_4$$

$$(VII);$$

$$Y=-2.9043+2.8920X_0-0.5471X_1-0.1049X_2+0.2281X_3-0.6194X_4$$

$$(VIII).$$

Example 3: Diagnosis of colorectal cancer

Totally 40 patients with colorectal cancer and adenomatous polyps and 18 healthy people were selected and screened using the kit provided by the present invention, and sensitivity, specificity and accuracy of the present kit for colorectal cancer detection were judged according to the detection results.

1. Collection of fecal sample collection

Two sampling tubes were labeled as tube a and tube b, and one spoon of leveled feces (about 0.25 mL, 2g) was collected from the subjects and transferred into the tubes.

2. Fecal occult blood test

The tube a was used for the fecal occult blood testing in an automatic fecal treatment and analysis system according to the instructions of the fecal occult blood test kit (colloidal gold method). If the control line band and the test line band were positive, the result was positive and recorded as $X_0 = 1$. If the control line band was positive and the test line band was negative, the result was negative and recorded as $X_0 = 0$.

3. Detection of fecal biomarkers

3.1 Extraction of fecal genomic DNA

(1) 180-220 mg of the fecal sample in the above-mentioned tube b was weighed and transferred into a 2 mL centrifugal tube, and genomic DNA was extracted according to the instructions of the genomic extraction reagent.
(2) 180-220 mg of the fecal sample (or 200 $\mu$L of a liquid sample) was transferred into a 2 mL centrifugal tube, and the tube is placed on ice.
(3) 500 $\mu$L of glucose and EDTA buffer, 100 $\mu$L of lysate, 15 $\mu$L of Proteinase K and 0.25 g of milling balls were added to the sample, and shaken intermittently for 1 min until the sample was fully mixed; and incubated at 95 °C for 15 min, during which shaken 2-3 times.
(4) The mixture was oscillated for 15 s in a vortex oscillator and centrifuged at 12,000 rpm for 3 min. The supernatant was transferred into another centrifugal tube, to which 10 $\mu$L of RNase A was added, shaken to mix and then left to stand at room temperature for 5 min.
(5) 200 $\mu$L of NaOH-SDS buffer was added, shaken to mix and placed on ice for 5 min. The mixture was centrifuged at 12,000 rpm for 3 min, the obtained supernatant was transferred to another 1.5 mL centrifugal tube, to which the same volume of sodium acetate solution was added, and then transferred to an adsorption column CR2 (the adsorption column would be placed in a collecting tube). The adsorption column CR2 was centrifuged at 12,000 rpm for 30 s, and then placed in the collecting tube after pouring off the waste liquid.
(6) 500 $\mu$L of absolute ethanol was added to the adsorption column CR2, centrifuged at 12,000 rpm for 30 s, and the adsorption column CR2 was placed in the collecting tube after pouring off the waste liquid. 700 $\mu$L of rinsing liquid was added to the adsorption column CR2, centrifuged at 12,000 rpm for 30 s, and the adsorption column CR2 was placed in the collecting tube after pouring off the waste liquid, and the operation was repeated.

(7) The adsorption column CR2 was placed in the collecting tube, centrifuged at 12,000 rpm for 2 min, and then the waste liquid was pouring off. The adsorption column CR2 was left at room temperature for several minutes to completely dry the residual rinsing liquid in the adsorption material.

(8) The concentration and purity were detected with an ultraviolet spectrophotometer, where, the ratio of OD260/OD280 should be 1.7-1.9. The optimal concentration was $\geq$ 50 ng/$\mu$L to ensure that the extracted DNA was free of pollution. In this way, the fecal genomic DNA was obtained.

3.2 Real-time qPCR detection

(1) Preparation of real-time PCR solution: The solution was prepared on ice, and every 10 $\mu$L of the reaction system contained 5 $\mu$L of 2$\times$Mix; 0.3 $\mu$L of sense primer; 0.3 $\mu$L of anti-sense primer; 0.2$\mu$L of 50$\times$ROX; 3.2$\mu$L of RNase-free ddH$_2$O; and 20 ng of genomic DNA (40 ng, 20 ng, 10 ng and 5 ng of genomic DNA were added respectively when a standard curve was constructed).

(2) The experiment was carried out on a qPCR amplifier under the reaction conditions of 95 °C for 15 min; 45 cycles (95 °C, 10 s; 58/60 °C, 60 s or 95 °C, 10 s; 58/60°C 20s,72°C 30 s);1.6°C/s(95°C,15s;60°C,60s).Where, the primer pair (1) was annealed at 56 °C by a two-step process; the primer pair (2) was annealed at 58 °C by a two-step process; the primer pair (3) was annealed at 56 °C by a three-step process; the primer pair (4) was annealed at 60 °C by a three-step process; the primer pair (5) was annealed at 60 °C by a two-step process.

(3) A standard curve was drawn to calculate the amplification efficiency with the formula: efficiency = 10(-1/ slope)-1, and the efficiency was qualified within 90%-110%.

(4) The sample was tested according to the above steps, the primer-amplified value Ct$_{marker}$ corresponding to the biomarkers and the primer-amplified value Ct$_{total}$ bacteria of the total bacteria were recorded, and the cycle value Ct of each biomarker was calculated, where Ct = lg(2^(Ct$_{marker}$-Ct$_{total\ bacteria}$)).

4. Interpretation of diagnostic results

A value P was obtained with the following formula, and when P>0.5, the result was judged as positive; and when P$\leq$0.5, the result was judged as negative.

$$P = \frac{e^Y}{1+e^Y}$$

Wherein, Y=-3.9583+3.1015X$_0$-0.6478X$_1$-0.3980X$_2$+0.2912X$_3$-0.5130X$_4$, X$_0$ is an occult blood result value, X$_1$ is a Ct value of *Peptostreptococcus stomatis*, X$_2$ is a Ct value of *Fusobacterium nucleatum*, X$_3$ is a Ct value of *Parvimonas micra*, and X$_4$ is a Ct value of *Bifidobacterium*.

When P>0.5, the result was judged as positive. That is, it is confirmed that the subject had colorectal cancer. When P$\leq$0.5, the result was judged as negative. That is, it is confirmed that the subject did not have colorectal cancer.

5. Results analysis

The experimental results show that the present kit has high accuracy, specificity and sensitivity for diagnosis of colorectal cancer.

The above description is intended to be a detailed description of preferred and feasible examples of the present invention and is not intended to limit the scope of the present invention. Any equivalent change or modification made within the technical spirit suggested by the present invention shall fall within the scope of the present invention.

**Claims**

1. A biomarker combination for colorectal cancer, wherein the biomarker combination comprises *Peptostreptococcus stomatis, Fusobacterium nucleatum, Parvimonas micra* and *Bifidobacterium.*

2. A kit for detecting colorectal cancer, wherein the kit comprises an assay reagent for the biomarker combination according to claim 1, and the assay reagent is used for quantifying each component of the biomarker combination of claim 1.

**3.** The kit according to claim 2, wherein the principle followed by the assay reagent is real-time quantitative PCR.

**4.** The kit according to claim 3, wherein the assay reagent comprises a primer group for amplifying the biomarker combination according to claim 1, and the primer group comprises a primer pair for amplifying each component of the biomarker combination of claim 1.

**5.** The kit according to claim 4, wherein the primer group has nucleotide sequences as shown in SEQ ID NO. 1-8.

**6.** The kit according to claim 5, wherein the assay reagent further comprises a primer pair for amplifying total bacteria.

**7.** The kit according to claim 6, wherein the primer pair for amplifying the total bacteria has nucleotide sequences as shown in SEQ ID NO.9-10.

**8.** The kit according to claim 7, wherein the assay reagent further comprises a real-time quantitative PCR reagent.

**9.** The kit according to any of claims 2-8, wherein the kit further comprises an occult blood test reagent for detecting human hemoglobin in feces.

**10.** A use of a kit according to claim 9 in colorectal cancer early diagnosis, wherein colorectal cancer detection using the kit comprises the following steps: detecting an abundance and a fecal occult blood value of each component of a biomarker combination according to claim 1 in a sample, and then judging the detection result using a regression equation.

**11.** The use according to claim 10, wherein colorectal cancer detection using the kit comprises the following steps:

(1) determination of the fecal occult blood value: determining the occult blood result value $X_0$ of the fecal sample of a subject using the fecal occult blood test kit, where $X_0 = 1$ when the occult blood result is positive and $X_0 = 0$ when the occult blood result is positive;
(2) using genomic DNA from the feces of the subject as a template, detecting $Ct_{marker}$, an amplified value of each biomarker and $Ct_{total\ bacteria}$, an amplified value of the total bacteria with an assay reagent in the kit, separately;
(3) calculating a relative content (a Ct value) of each component of the biomarker combination in the total bacteria using the sample according to formula (I), separately,

$$Ct=lg(2^{\wedge}(Ct_{marker}-Ct_{total\ bacteria}))$$

$$(I);$$

(4) calculating a value Y according to a regression equation, and substituting the value Y into formula (II) to calculate a value P, where e is a natural constant,

$$P=\frac{e^{Y}}{1+e^{Y}}$$

$$(II);$$

(5) interpretation of result: when $P>0.5$, the diagnosis result of colorectal cancer is positive; and when $P\leq0.5$, the diagnosis result of colorectal cancer is negative.

**12.** The use according to claim 11, wherein the regression equation in step (4) is shown as formula (III),

$$Y=A+\beta_0 X_0+\beta_1 X_1+\beta_2 X_2+\beta_3 X_3+\beta_4 X_4$$

$$(III);$$

where, $X_0$ is the occult blood result value, $X_1$ is a Ct value of *Peptostreptococcus stomatis,* $X_2$ is a Ct value of *Fusobacterium nucleatum,* $X_3$ is a Ct value of *Parvimonas micra* and $X_4$ is a Ct value of *Bifidobacterium;* A and $\beta_0$-$\beta_4$ are constants, and A and $\beta_0$-$\beta_4$ are data obtained from clinical experiments.

**13.** The use according to claim 12, wherein the regression equation in step (4) comprises any of formula (IV) to formula (VIII):

$$Y=-3.9583+3.1015X_0-0.6478X_1-0.3980X_2+0.2912X_3-0.5130X_4 \quad \text{(IV)};$$

$$Y=-2.965+2.7005X_0-1.2309X_1-0.6054X_2+0.3328X_3-0.2359X_4 \quad \text{(V)};$$

$$Y=-3.099+1.9907X_0-2.0011X_1-0.3290X_2+0.5476X_3-0.6620X_4 \quad \text{(VI)};$$

$$Y=-3.771+4.0089X_0-0.9983X_1-0.4482X_2+0.1288X_3-0.4917X_4 \quad \text{(VII)};$$

$$Y=-2.9043+2.8920X_0-0.5471X_1-0.1049X_2+0.2281X_3-0.6194X_4 \quad \text{(VIII)}.$$

## INTERNATIONAL SEARCH REPORT

| International application No. |
| --- |
| **PCT/CN2022/087384** |

**A. CLASSIFICATION OF SUBJECT MATTER**

C12Q 1/689(2018.01)i; C12Q 1/06(2006.01)i; G06F 17/18(2006.01)i

According to International Patent Classification (IPC) or to both national classification and IPC

**B. FIELDS SEARCHED**

Minimum documentation searched (classification system followed by classification symbols)

C12Q; G06F

Documentation searched other than minimum documentation to the extent that such documents are included in the fields searched

Electronic data base consulted during the international search (name of data base and, where practicable, search terms used)

CNABS, DWP, WPABSC, CNTXT, ENTXTC, ENTXT, OETXT, WPABS, VCN, VEN, CNKI, 百度学术搜索, BAIDU SCHOLAR SEARCH, WEB OF SCIENCE, PubMed: 结直肠癌, colorectal carcinoma, colorectal cancer, 口炎消化链球菌, Peptostreptococcus stomatis, 具核梭状杆菌, Fusobacterium nucleatum, 微小微单胞菌, Parvimonas micra, 双歧杆菌, Bifidobacterium, 隐血, occult blood; GenBank, EMBL, 中国专利生物序列检索系统, Chinese Patent Biological Sequence Retrieval System: SEQ ID NOs: 1-10

**C. DOCUMENTS CONSIDERED TO BE RELEVANT**

| Category* | Citation of document, with indication, where appropriate, of the relevant passages | Relevant to claim No. |
| --- | --- | --- |
| PX | CN 113106163 A (LANZHOU UNIVERSITY) 13 July 2021 (2021-07-13) <br> see claims 1-13 | 1-13 |
| X | CN 110512015 A (SUZHOU PRECISIONGENE TECHNOLOGY CO., LTD.) 29 November 2019 (2019-11-29) <br> see claims 1-9, and description, embodiment 2 | 1-8 |
| Y | CN 110512015 A (SUZHOU PRECISIONGENE TECHNOLOGY CO., LTD.) 29 November 2019 (2019-11-29) <br> see claims 1-9 | 9-13 |
| Y | CN 106399570 A (HANGZHOU NEW HORIZON HEALTH TECHNOLOGY CO., LTD.) 15 February 2017 (2017-02-15) <br> see claims 1-9 | 9-13 |
| A | US 2018122511 A1 (UBIOME, INC.) 03 May 2018 (2018-05-03) <br> see abstract | 1-13 |

☑ Further documents are listed in the continuation of Box C.  ☑ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **01 July 2022** | **22 July 2022** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/087384** |

**C.    DOCUMENTS CONSIDERED TO BE RELEVANT**

| Category* | Citation of document, with indication, where appropriate, of the relevant passages | Relevant to claim No. |
|---|---|---|
| A | US 2021000885 A1 (KOVARIK, J. E.) 07 January 2021 (2021-01-07) see abstract | 1-13 |
| A | 王赫等 (WANG, He et al.). "肠道菌群与结直肠肿瘤关系的研究进展 (Research Progress in Relationship between Gut Microbiota and Colorectal Cancer)" 中国普通外科杂志 (*Chinese Journal of General Surgery*), Vol. 29, No. 10, 25 October 2020 (2020-10-25), pp. 1261-1269, see entire document | 1-13 |

**INTERNATIONAL SEARCH REPORT**

| International application No. |
|---|
| **PCT/CN2022/087384** |

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

 a.  ☑ forming part of the international application as filed:

   ☑ in the form of an Annex C/ST.25 text file.

   ☐ on paper or in the form of an image file.

 b.  ☐ furnished together with the international application under PCT Rule 13ter.1(a) for the purposes of international search only in the form of an Annex C/ST.25 text file.

 c.  ☐ furnished subsequent to the international filing date for the purposes of international search only:

   ☐ in the form of an Annex C/ST.25 text file (Rule 13ter.1(a)).

   ☐ on paper or in the form of an image file (Rule 13ter.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

INTERNATIONAL SEARCH REPORT
Information on patent family members

International application No.

**PCT/CN2022/087384**

| Patent document cited in search report | | | Publication date (day/month/year) | Patent family member(s) | | | Publication date (day/month/year) |
|---|---|---|---|---|---|---|---|
| CN | 113106163 | A | 13 July 2021 | None | | | |
| CN | 110512015 | A | 29 November 2019 | WO | 2021047019 | A1 | 18 March 2021 |
| CN | 106399570 | A | 15 February 2017 | None | | | |
| US | 2018122511 | A1 | 03 May 2018 | None | | | |
| US | 2021000885 | A1 | 07 January 2021 | None | | | |

Form PCT/ISA/210 (patent family annex) (January 2015)

**REFERENCES CITED IN THE DESCRIPTION**

**Patent documents cited in the description**

- CN 106574294 A
- CN 110512015 A